# EUROPEAN PATENT APPLICATION

(11) **EP 3 412 764 A1**
(43) Date of publication of application: **12.12.2018**
(21) Application number: 17174846.0
(22) Date of filing: 07.06.2017
(51) Int. Cl.: C12M 3/06, C12M 1/02, C12M 1/42, C12M 1/00, B01L 3/00

(54) **MICROFLUIDIC DEVICE**

(71) Applicant: Leibniz - Institut für Analytische Wissenschaften - ISAS - e.V., 44139 Dortmund (DE)
(72) Inventor: NOVO, Pedro, 45966 Gladbeck (DE); DELL'AICA, Margherita, 44137 Dortmund (DE); JANASEK, Dirk, 44139 Dortmund (DE); ZAHEDI, René, 44139 Dortmund (DE)
(74) Representative: Simandi, Claus

(57) **Abstract**

A microfluidic device for mixing fluids and controlled stimulation of cells with fast temporal resolution, comprising a first mixing device for mixing cell suspension and stimulant medium connected downstream to a second mixing device for mixing the output of the first mixing device with quenching medium.

## Description

The present invention relates to the field of controlled cell stimulation *in vitro* and provides a microfluidic device for mixing a suspension of cells with a stimulant and thus means and methods for controlled cell stimulation.

Responses of biological cells to external and in particular chemical stimuli may occur within only a few seconds or even in the range of milliseconds, for example, protein phosphorylation upon ligand/cell receptor interaction. For example, upon cell ligand/membrane receptor interaction, the phosphorylation of the receptor and the following signaling cascade is executed within milliseconds. Precise studies on such interactions thus require a very high temporal resolution of the stimulation regimen and of the analysis sampling as well.

However, conventional cell stimulation methods, based on mixing or switching of media only achieve effective time resolutions in the range of 5 to 60 seconds. Although microfluidic devices using pinched flow (STE) strategy have already been employed to control fast cell stimulation with high temporal resolution, they are applicable only to certain cell types because of the dependency of STE on cell size and shape and also require long experiments (hours) to collect relevant amounts of cell products after stimulation, which most compromises cell integrity.

Thus, means and methods are desirable with stimulation of cells with stimulant compounds at high temporal resolution which allow for the collection of sufficient amounts of cell products in short time after stimulation, the application to any non-adherent cell type, and the avoidance of high flow rates which require high amounts of stimulant compounds and/or cells.

The present invention provides, in a first aspect, a microfluidic device for the controlled stimulation of cells with at least one stimulant, wherein the cells are preferably provided in a suspension and the stimulant is preferably provided dissolved in a medium. The device of the invention comprises a, preferably one, first micromixer device which has an, preferably one, first inlet for cells, i.e. cell suspension, and at least one, preferably two, inlets for stimulant, i.e. stimulant medium, and a plurality of mixing stages for mixing cells and stimulant to result in a first mix, and an outlet for said first mix. According to the invention, the microfluidic device further comprises a transfer channel which is arranged downstream the first micromixer device and in fluid connection with the outlet of the first micromixer device to receive and to transfer said first mix to a second micromixer device downstream the transfer channel.

The device of the present invention thus further comprises a second micromixer device arranged downstream having an, preferably one, inlet for the first mix received through the transfer channel and at least one, preferably two inlets for a quencher, i.e. a quencher comprising medium. The second micromixer device also comprises a plurality of mixing stages for mixing said first mix and the quencher to result in a second mix. The second micromixer device further comprises an outlet for said second mix.

In a preferred embodiment, the device further comprises a sample collector arranged downstream of the second micromixer device for collecting the second mix from the outlet.

By means of this arrangement, it is possible to precisely control the periods of time for mixing the stimulant with the cells (t1), the stimulation time (t2), and the time for quenching and eventually lysis of the stimulated cells after stimulation (t3) at high temporal resolution. The suspension of quenched and eventually lysed cells can be collected downstream of the microfluidic device in a sample collector for further analysis at high temporal resolution of the samples. The present invention allows for low flow rates, thus for the saving of substantial amounts of cells and stimulant compound as compared to known devices for mixing and switching of media. The microfluidic device of the present invention allows for substantially fast and accurate and controllable stimulation of suspended cells at high temporal resolution in the millisecond range.

Adjustment of mixing time (t1) can easily be achieved by selecting the dimensions (at a given flow rate) and/or number of mixing stages in the first micromixer device. For example, each mixing stage can be designed to represent about 20 ms of mixing time. A four-staged device, which is a preferred embodiment, thus would provide for 80 ms mixing time. That is, cells and stimulant are fully mixed in this embodiment after 80 ms.

Adjustment of stimulation time (t2) can easily be achieved by selecting the dimensions, specifically length and/or diameter (at a given flow rate) of the transfer channel between the first micromixer device and the second micromixer device. In preferred embodiments, the controlled time stimulation ranges from about 100 ms to 10 seconds.

Adjustment of quenching time (t3) can easily be achieved by selecting the dimensions (at a given flow rate) and/or number of mixing stages in the second micromixer device. For example, each mixing stage can be designed to represent about 20 ms of mixing time. A four-stage device, which is a preferred embodiment, thus would provide for 80 ms quenching time. That is, stimulated cells and quenching agent are fully mixed in this embodiment latest after 80 ms.

In a preferred embodiment, not only quenching of the stimulation takes place in the second micromixer device, but also lysis of the cells, to facilitate the release of cell products or compounds from the stimulated cells for analysis.

In the context of the present invention, a "quencher" thus not only refers to a compound which quenches the stimulation introduced by the stimulant, but a quencher may also comprise at least one agent capable of effecting cell lysis.

In the context of the present invention, a "stimulant" refers to any chemical compound which may be present in liquid medium which has effect on the cells' integrity, function, and more particular physiological reactions. A stimulant may be selected from pharmaceuticals, caustic agents or solvents, as well as from growth factors, stimulating factors, cytokines, hormones, neurotransmitters, electrolytes, such as potassium, sodium, calcium, magnesium, blockers to ion channels or to transmembrane transport, sugars, proteins, glycoproteins, amino acids, other phosphorylating agents, DNA, RNA, specifically mRNA, viruses and fragments thereof, biological cells and fragments thereof, immunostimulating agents and antigens, as well as nanoparticles specifically nanodrugs.

In a preferred embodiment, the first micromixer device comprises 2 to 6 mixing stages, preferably 3 to 5, more preferred 4 mixing stages.

In a preferred embodiment, the second micromixer device comprises 2 to 6 mixing stages, preferably 3 to 5, more preferred 4 mixing stages.

In a preferred embodiment, the transfer channel is adjustable in length. In a preferred embodiment the transfer channel is adjustable in diameter. This allows for precise adjustment and control of the stimulation time (t2) after mixing in the first micromixer device and before quenching in the second micromixer device.

In preferred embodiments, the device may include further micromixer devices in a consecutive row to bring about a series of stimulation levels. Each micromixer device is designed to provide a specific level, i.e. concentration, of stimulant to the cells. The last micromixer devices in the row, downstream the preceding devices may introduce the quencher and/or lysis to the cells, thereby ending the controlled stimulation. The series of micromixer devices may also introduce various stimulants in a specific order in downstream direction of cell flow, such as to provide a predetermined stimulation regimen. The consecutively applied stimulants introduced by the one or more further micromixer devices may include, but are not limited to, agonists, antagonists, co-factors, modulators or inhibitors to the stimulant initially added in the first micromixer devices.

In a second aspect, the present invention provides for a method for controlled stimulation of cells with a stimulant for a particular period of time at high temporal resolution, the method comprising the following steps: In a first step a liquid suspension of cells is provided and a liquid medium containing the stimulant is provided; in a next step stimulant medium and suspended cells mixed in a first micromixer device during a first period of time (t1) to obtain a first mix, comprising the suspended cells and the stimulant. In a preferred variant t1 is from 20 ms to 100 ms, preferably 40 ms to 80 ms, more preferred 80 ms.

In the next step, the first mix obtained from the first micromixer device is transferred to a second micromixer device via a transfer channel during a second period of time (t2). Time t2 corresponds to the controlled time for the stimulation of the cells with the stimulant and is preferably selected according to the present invention by adjusting the length and/or the diameter of the transfer channel (at a given flow rate of the first mix in the transfer channel). In a preferred variant t2 is from 100 ms to 10 seconds. In the Senate applications it is preferred that t2>>t1 and more preferably also t2>>t3.

Then a liquid medium containing a quencher and eventually a separate lysis agent is provided, and the quenching medium and the first mix obtained from the transfer channel is mixed in a second micromixer device during a third period of time (t3) to obtain a second mix comprising the suspended cells after quenching, and eventually lysed cells after lysis. Quenching of the stimulation and eventually lysis of the cells takes place in the second micromixer device. In a preferred variant t3 is from 20 ms to 100 ms, preferably 40 ms to 80 ms, more preferred 80 ms.

The resulting second mix is preferably collected and sampled in a collecting device or sample collector downstream, and most preferred directly from, the second micromixer device for analysis with high temporal resolution of the samples.

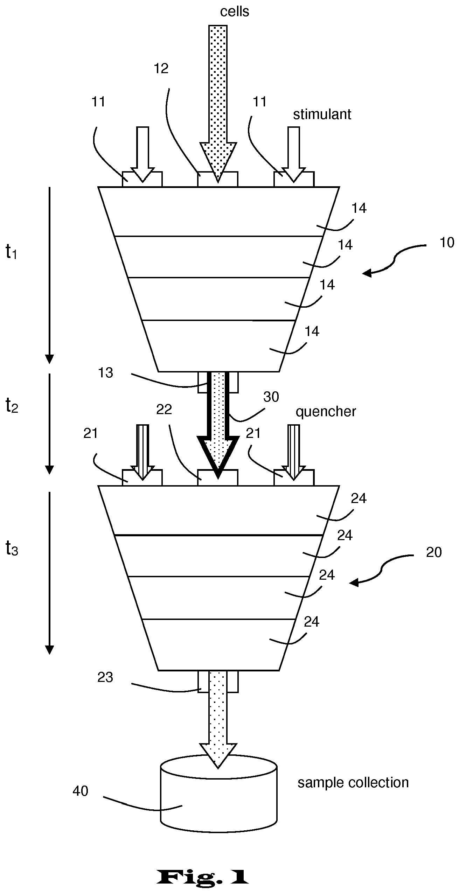

Figure 1 depicts a schematic view of the microfluidic device of the present invention. A first micromixer device 10 comprises at least one inlet 11 for stimulant media and one inlet 12 for cell suspension. In four consecutive mixing stages 14 stimulant and cell suspension can be fully mixed leaving the first micromixer device 10 via outlet 13 opening into a transfer channel 30. The mixture of stimulant and cells is transferred via the transfer channel 30 to a second micromixer device 20 the second micromixer device 20 comprises at least one inlet 21 four quenching media and one inlet 22 for the mix leaving the first micromixer device 10. In four consecutive mixing stages 24 quencher and cell suspension mix can be fully mixed leaving the second micromixer device 20 via outlet 23. In the depicted embodiment, outlet 23 opens to a sample collector 40 for sampling the cell suspension after stimulation. The period of time for mixing the stimulant with the cells (t1) is particularly determined by the design of the first micromixer device 10. The stimulation time (t2) is specifically determined by the design of the transfer channel 30, more specifically by the length and the flash on the diameter of the channel. The time for quenching and eventually lysis of the cells after stimulation (t3) is particularly determined by the design of the second micromixer device 20.

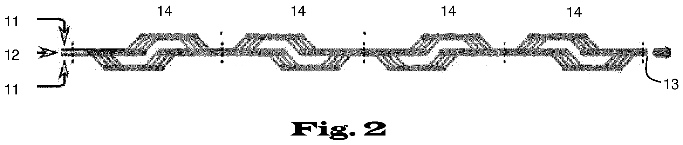

Figure 2 schematically shows the basic structure of the micromixer device 10 having two inlets 11 for stimulant, one inlet 12 for cell suspension, four mixing stages 14 and an outlet 13 for the mix. This is a 3-inlet, 1-outlet device.

### EXAMPLE

The device is composed of two mixing segments connected in series: the first for mixing of cells with stimulation solution and the second for cell lysis. Different stimulation time points were achieved by varying the dead volume between first and second mixing segments at constant flow rates. Each mixing stage is comprised of a two-level fluidic network with three inlets and one outlet. The top level includes the main channel network (channels with 350×160 µm² cross-section). The bottom level contains a series of smaller cross-section channels (125×50 µm²) interconnecting the main channels.

The microfluidic device was fabricated in a two-level PDMS configuration via soft-lithography. PDMS (Elastosil RT 601 A/B, Wacker, Germany) was mixed at a 10:1 ratio. The bottom level was fabricated by spin-coating the PDMS on a SU8 master on a 6" silicon wafer to a final thickness of 1 mm and cured at 100 °C for 15 min. Next, corona discharge was applied to both microscope glass slides (1"×3") and PDMS on top of the wafer. The glass slides were aligned with the microfluidic structures, bonded and let to rest for 1 h. The top PDMS level was fabricated by pouring PDMS inside a 4 mm thick PMMA frame aligned with the SU8 microfluidic structures on a 6" silicon wafer and cured at 80 °C for 1 h. Next, inlet/outlet holes were punched into the top PDMS layer using a 3 mm Ø biopsy puncher. Then, O₂ plasma was applied to both PDMS layers which were aligned manually and let to bond for at least 3 h. Silicon tubing 3 mm OD was inserted into the inlet/outlets holes, sealed with silicone (Elastosil E41, Wacker) and let to dry overnight.

Teflon tubing (1.6 mm OD) was connected directly to the silicone tubing and to syringes mounted on a syringe pump (neMESYS, cetoni, Germany) for fluid flow control. In particular, a temperature control device with magnetic stirring capability, developed in-house, was used to maintain cells homogeneously distributed inside the syringe, using 3 mm diam. magnetic beads, and at 37 °C.

Experiments were performed using HEK cells that overexpressed the EGF receptor to study early time points of phosphorylation-based signaling upon EGF stimulation. Cell stimulation and lysis were carried out in microfluidics using the two-segment micromixer device to ensure reproducibility. Cells treated with buffer in absence of EGF served as control. After stimulation cells were collected in lysis buffer and subjected to a highly sensitive and accurate quantitative phosphoproteomics workflow. Label free LC-MS and parallel reaction monitoring (PRM) was applied to quantify proteins and phosphopeptides. Around 80 µg of peptides were separated for phosphopeptide enrichment using TiO₂ beads. Different stimulation time points were achieved by utilizing differently dimensioned microfluidic devices where the dead volume between mixing segments was adapted to obtain the desired duration values.

It was possible to simultaneously quantify EGF-dependent changes in the phosphorylation of 15 different proteins. PRM allowed precise quantification of changes across the different conditions. Results demonstrate that EGFR auto-phosphorylation occurs already after 500 ms while most downstream signaling proteins show initial changes after 1 to 5 seconds.

FITC and Rhodamine B containing solutions were used to demonstrate the mixing efficiency as a function of the micromixer location. Four mixing stages ensure complete mixing of the solutions with the experimental data fitting well the simulated results. At the 4^{th} mixing stage transversal cross-section, the fluorescence coefficient of variation is only 7 % (vs 3 % theoretically obtained).

### REFERENCE LIST

- 10: first micromixer device
- 11: cell inlet
- 12: stimulant inlet
- 13: first mix outlet
- 14: mixing stage
- 20: second micromixer device
- 21: mix inlet
- 22: quencher inlet
- 23: second mix outlet
- 24: mixing stage
- 30: transfer channel
- 40: sample collector

## Claims

1. A microfluidic device for controlled stimulation of cells with a stimulant, comprising:
- a first micromixer device (10), having a first inlet (11) for the cells and at least one inlet (12) for the stimulant, multiple mixing stages (14) for mixing cells and stimulant to a first mix, and an outlet (13) for said first mix;
- a transfer channel (30) downstream the first micromixer device (10) in fluid connection to the mix outlet (13); and
- a second micromixer device (20), downstream the transfer channel (30) having a first inlet (21) in fluid connection to the transfer channel (30) for said first mix and at least one inlet (22) for a quencher, multiple mixing stages (24) for mixing first mix and quencher to a second mix, and an outlet (23) for said second mix.

2. The device of claim 1, further comprising a sample collector (40) downstream the second micromixer device (20) for collecting the second mix from outlet (23).

3. The device of claim 1 or 2, wherein the first micromixer device (10) comprises two to six mixing stages (14).

4. The device of any one of the preceding claims, wherein the second micromixer device (10) comprises two to six mixing stages (14).

5. The device of any one of the preceding claims, wherein the transfer channel (30) is adjustable in diameter and/or length.

6. The device of any one of the preceding claims, further comprising:
- one or more further micromixer devices and transfer channels arranged in-between the first micromixer device (10) second micromixer device (20), for a series of controlled stimulations of the cells with stimulants.

7. A method for controlled stimulation of cells with a stimulant, comprising the steps of:
- providing a liquid suspension of cells,
- providing a liquid medium containing the stimulant
- mixing stimulant medium and suspended cells in a first micromixer device (10) in a first period of time **t₁** to obtain a first mix, comprising suspended cells,
- transferring said first mix to a second micromixer device (20) via a transfer channel (30) in a second period of time **t₂,**
- providing a liquid medium containing a quencher;
- mixing quenching medium and first mix in said second micromixer device (20) in a third period of time **t₃** to obtain a second mix, comprising suspended cells.

8. The method of claim 7, further comprising the step of:
- collecting and sampling the second mix from said second micromixer device (20)

9. The method of claim 7 or 8, wherein **t₂** corresponds to the the controlled time for the stimulation of the cells with the stimulant.

10. The method of claim 9, wherein **t₂** is selected by adjusting length and/or diameter of the transfer channel (30) at a given flow rate of the first mix.
